(19) Europäisches Patentamt
European Patent Office
Office européen des brevets

(11) Veröffentlichungsnummer : **0 014 911**
**B1**

(12) # EUROPÄISCHE PATENTSCHRIFT

(45) Veröffentlichungstag der Patentschrift : **26.01.83**

(51) Int. Cl.[3] : **C 07 C103/52**, A 61 K 37/43

(21) Anmeldenummer : **80100635.4**

(22) Anmeldetag : **07.02.80**

(54) **Dipeptid (pyro)Glu-His(Dnp)-OH und Verfahren zur Herstellung von LH-RH und LH-RH-Analoga unter Verwendung dieses Dipeptids.**

(30) Priorität : **14.02.79 DE 2905502**

(43) Veröffentlichungstag der Anmeldung : **03.09.80 Patentblatt 80/18**

(45) Bekanntmachung des Hinweises auf die Patenterteilung : **26.01.83 Patentblatt 83/04**

(84) Benannte Vertragsstaaten : **AT BE CH DE FR GB IT LU NL SE**

(56) Entgegenhaltungen :
**BE A 869 076**
**DE A 2 830 629**
**FR A 2 183 021**
**FR A 2 198 931**
**FR A 2 248 267**
**FR A 2 270 890**
**FR A 2 282 907**
**FR A 2 313 940**
**FR A 2 347 335**
**US A 3 931 138**
**US A 3 959 248**

(73) Patentinhaber : **HOECHST AKTIENGESELLSCHAFT**
**Postfach 80 03 20**
**D-6230 Frankfurt/Main 80 (DE)**

(72) Erfinder : **König, Wolfgang, Dr.**
**Eppsteiner Strasse 25**
**D-6238 Hofheim am Taunus (DE)**
Erfinder : **Teetz, Volker, Dr.**
**Rebhuhnweg 11**
**D-6238 Hofheim am Taunus (DE)**
Erfinder : **Jäger, Georg, Dr.**
**Am Rehsteig 9**
**D-6232 Bad Soden am Taunus (DE)**
Erfinder : **Geiger, Rolf, Dr.**
**Heinrich-Bleicher-Strasse 33**
**D-6000 Frankfurt am Main 50 (DE)**

EP 0 014 911 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Dipeptid Pyro Glu-His(Dnp)-OH und Verfahren zur Herstellung von LH-RH und LH-RH-Analoga unter Verwendung dieses Dipeptids

Gegenstand der Erfindung ist ein Verfahren zur Herstellung von LH-RH bzw. LH-RH-Analoga, das dadurch gekennzeichnet ist, daß man <Glu-His(Dnp)-OH mit dem entsprechenden Peptid mit freier Amino- und geschützter Carboxylgruppen in einem in der Peptidchemie üblichen Lösungsmittel unter Zusatz von 3-Hydroxy-4-oxo-3.4-dihydro-1.2.3-benzotriazin (HOObt) und einem Carbodiimid umsetzt und anschließend die Dnp-(2.4-Dinitrophenyl)-gruppe abspaltet.

LH-RH ist bekanntlich ein Hormon aus dem Hypothalamus der Formel I

$$\text{<Glu-His-Trp-Ser-Tyr-Gly-Leu-Arg-Pro-Gly-NH}_2, \qquad (I)$$

das in der Hypophyse die gonadotropen Hormone LH und FSH ausschüttet. Als LH-RH-Analoga kommen Peptide in Betracht, in denen einzelne oder mehrere Aminosäuren des LH-RH ausgetauscht sind und/oder die Peptidkette durch Verkürzung, Verlängerung und/oder Derivatisierung variiert ist. Von besonderer Bedeutung sind hierbei Substitutionen von Glycin in Position 6 durch D-Aminosäuren und in Position 10 durch Alkylamine.

Als zweite Komponente für das erfindungsgemäße Verfahren kommen somit vor allem Octa- bzw. Heptapeptide der allgemeinen Formel II oder III

$$\text{II : H-Tryp-Ser-Tyr-X-Leu-Arg-Pro-Gly-NH}_2$$

$$\text{III : H-Trp-Ser-Tyr-X-Leu-Arg-Pro-NH-C}_2\text{H}_5$$

infrage, in denen X Gly oder D-Aminosäuren bzw. deren Derivate wie z.B. D-Ser($Bu^t$), D-Leu, D-Ala, D-Phe, D-Trp, D-Gln(cyclohexyl), D-Glu($OBu^t$) und D-Lys(Boc) bedeuten.

Das als Ausgangsstoff verwendete neue Dipeptidderivat <Glu-His(Dnp)-OH wird in üblicher Weise, z.B. durch Umsetzung von <Glu-His-OH mit 2.4-Dinitrofluorbenzol in wasserhaltiger, mit $NaHCO_3$ abgepufferten Lösung hergestellt.

Als geeignetes Lösungsmittel verwendet man bei dem erfindungsgemäßen Verfahren aus Gründen der Löslichkeit polare Lösungsmittel wie z.B. Dimethylacetamid, Dimethylformamid, Dimethylsulfoxid, Phosphorsäure-tris-(dimethylamid) oder N-Methyl-pyrrolidon.

Die Synthese kann zwischen – 10 °C und Raumtemperatur durchgeführt werden. Vorzugsweise beginnt man bei ca. 0 °C und läßt später auf Raumtemperatur kommen.

Als Kondensationsmittel wird das leicht zugängliche Dicyclohexylcarbodiimid (DCC) bevorzugt, doch auch andere Carbodiimide wie z.B. 1-Cyclohexyl-3-(2-morpholino-äthyl)-carbodiimid-toluolsulfonat oder 1-Äthyl-3-(3-dimethyl-aminopropyl)-carbodiimid-hydrochlorid sind verwendbar. Als Zwischenprodukte entstehen zunächst Peptide, die am Imidazolring des Histidins durch den Dinitrophenylrest geschützt sind. Dieser Dinitrophenylrest kann nach in der Peptidchemie bekannten Methoden abgespalten werden. Geeignete Abspaltungsmethoden sind beispielsweise die Thiolyse (Biochem-Biophys. Res. Commun. *29* 178 (1967) ; Biochemistry *9*, 5122 (1970)) oder die Hydrazinolyse (Tetrahedron Letters *44*, 4121 (1971)).

Das Dnp-haltige Zwischenprodukt braucht im Allgemeinen nicht isoliert zu werden. Nach der Peptid-Kupplung gibt man zum Reaktionsansatz z.B. Thiole wie Mercaptoäthanol oder Äthylmercaptan bzw. Hydrazin und arbeitet nach vollzogener Abspaltung des Dnp-Restes, was durch dünnschichtchromatographische Kontrolle leicht festgestellt werden kann, den Ansatz auf. Wird Hydrazin als Deblockierungsreagens verwendet, ist Dimethylformamid als Lösungsmittel nicht geeignet, weil es gegenüber Hydrazin nicht stabil ist.

Es ist bereits bekannt, daß sich zur Herstellung von LH-RH sowie dessen Analoga besonders die Verknüpfung von <Glu-His-OH mit den entsprechenden Peptiden mittels DCC unter Zusatz von racemisierungssenkenden N-Hydroxyverbindungen wie z.B. N-Hydroxysuccinimid (HONSu), 1-Hydroxybenzotriazol (HOBt), 3-Hydroxy-4-oxo-3,4-dihydro-1,2,3-benzotriazin (HOOBt) oder N-Hydroxy-5-norbornen-endo-2,3-dicarboximid (HONB) eignet, vgl. Biochem. Biophys. Res. Commun *45* 767-773 (1971).

Mittels Hochdruckflüssigkeitschromatographie (= HPLC = high pressure liquid chromatography) wurde jedoch festgestellt, daß bei diesem Verfahren die teilweise Racemisierung des Histidins nicht vermieden werden kann (siehe Tabelle 1 und 2). Zur Senkung der Racemisierung des Histidins bei der Synthese von Histidinpeptiden wird in der Literatur der Schutz des Imidazolringes durch die Tosylgruppe (= Tos-Gruppe) empfohlen. Entsprechender Schutz durch die 2.4-Dinitrophenylgruppe (= Dnp-Gruppe) scheint dagegen wegen zu starker Racemisierung des Histidins und Neigung zu weiteren Verunreinigungen weniger geeignet zu sein. (Rec. Trav. Chim. Pays-Bas *93*, 256 (1974)).

Es war daher überraschend, daß die Racemisierung des Histidins auf unter 2 % D-Histidin reduziert wird, wenn man in dem Ausgangsstoff <Glu-His-OH das Histidin durch die $N^{im}$ 2.4-Dinitrophenylgruppe (Dnp) schützt und die Reaktions-bedingungen des erfindungsgemäßen Verfahrens anwendet. Unter gleichen Bedingungen konnte dagegen mit der Tosylgruppe als $N^{im}$-Schutz die Racemisierung nicht unter 5 % D-Histidin gesenkt werden.

Vergleichende Untersuchungen bei der LH-RH-Synthese, ausgehend von verschiedenen Carboxylkomponenten unter Verwendung verschiedener Kondensationsmethoden sind in Tabelle 1 angegeben, hierbei ist mit Hilfe von Hochdruckflüssigkeitschromatographie (HPLC = high pressure liquid chromatography) der Gehalt an [D-His$^2$]-LH-RH bestimmt. Tabelle 1 zeigt, daß ausschließlich unter Verwendung der Methode DCC-HOObt und N$^{im}$-Dnp-Schutz die Racemisierung auf unter 2 % D-Histidin zurückgedrüngt werden kann.

Entsprechendes ergibt sich bei der Synthese eines LH-RH-Analogons, wie in Tabelle 2 angegeben.

Tabelle 1

Racemisierungsuntersuchungen bei der LH-RH-Synthese

Synthesebedingungen analog Beispiel 3-5.

HPLC : Säule (0.4 × 25 cm) gefüllt mit LiChrosorb(R)SI 60 der Firma Merck ; Elutionsmittel : 310 Chloroform, 190 Methanol, 14 Wasser, 3.1 Triäthylamin, 1 Ameisensäure (Volumeneinheiten) ; Elutionsgeschwindigkeit : 1 ml/Minute ;

$R_T$ (LH-RH) = ca. 14.0-14.5 Minuten

$R_T$ ([D-His$^2$]LH-RH) = Ca. 17.5-18 Minuten

| Carboxylcomponente | Methode | % [D-His$^2$] LH-RH |
|---|---|---|
| ⌜Glu-His-OH | DCC/HOBt | 23 |
| — | DCC/HONB | 15 |
| — | DCC/HONSu | 15 |
| — | DCC/HOObt | 12 |
| ⌜Glu-His(Tos)-OH | DCC/HONSu | 9 |
| — | DCC/HOObt | 5 |
| ⌜Glu-His(Dnp)-OH | DCC/HOBt | 25 |
| — | DCC/HONB | 7 |
| — | DCC/HONSu | 6 |
| — | DCC/HOObt | 1-2 |

Tabelle 2

Racemisierungsuntersuchungen bei der Synthese von [D-Ser(Bu$^t$)$^6$]LH-RH-(1-9)-nonapeptid-äthylamid

Synthesebedingungen analog Beispiel 6 und 7.

HPLC : Säule (0.4 × 25 cm) gefüllt mit LiChrosorb(R)SI 60 der Firma Merck ; Elutionsmittel : 410 Acetonitril, 29 Methanol, 29 Wasser, 20 Chloroform, 3.7 Triäthylamin, 1 Ameisensäure (Volumeneinheiten) ; Elutionsgeschwindigkeit : 2 ml/Minute ; $R_T$([D-Ser(Bu$^t$)$^6$]LH-RH-(1-9)-nonapeptid-äthylamid) = 40 Minuten, $R_T$([D-His$^2$, D-Ser(Bu$^t$)$^6$]LH-RH-(1-9)-nonapeptid-Äthylamid) = 48 Minuten

| Carboxylcomponente | Methode | % D-His$^2$ Verbindung |
|---|---|---|
| ⌜Glu-His-OH | DCC/HOBt | 20 |
| — | DCC/HONSu | 11 |
| — | DCC/HONB | 11 |
| — | DCC/HOObt | 6 |
| ⌜Glu-His-(Dnp)-OH | DCC/HOBt | 12 |
| — | DCC/HONSu | 4 |
| — | DCC/HONB | 3 |
| — | DCC/HOObt | 1-1,5 |

Beispiel 1

Herstellung von ⌜Glu-His(Dnp)-OH · 0.5 $H_2O$

30 g (100 mmol) ⌜Glu-His-OH · $H_2O$ und 20 g (238 mmol) $NaHCO_3$ werden in 200 ml Wasser gelöst. Dazu läßt man unter Rühren innerhalb einer Stunde eine Lösung von 22.3 g (120 mmol) 2.4-Dinitroflu-

*) LiChrosorb(R) ist ein Kieselgel mit mittlerer Porenweite von 60 Å.

orbenzol in 100 ml Dioxan zutropfen. Danach läßt man 3 Stunden bei Raumtemperatur rühren. Der Niederschlag wird abgesaugt und das Filtrat 2 mal mit je 200 ml Essigester extrahiert. Die wäßrige Phase wird eingeengt. Der Rückstand wird in 200 ml Wasser gelöst und mit 120 ml 1N HCl versetzt. Von ausfallendem roten Öl wird abdekantiert und filtriert. Zur wäßrigen Lösung gibt man 300 ml n-Butanol, schüttelt gut durch und läßt das Phasengemisch über Nacht bei 4 °C stehen. Anderntages wird der gelbe Niederschlag abgesaugt und mit wenig n-Butanol gewaschen. Der Niederschlag wird zur vollständigen Entfernung von noch vorhandenem <Glu-His-OH zwei mal mit je 300 ml Wasser eine Stunde bei Raumtemperatur verrührt, abgesaugt und über $P_2O_5$ i. Vak. getrocknet.

Ausbeute : 20 g
Schmp. 235-241 °C unter Zersetzung
$[\alpha]_D^{21} = -12.2$ °C (c = 1, in Essigsäure)
$[\alpha]_D^{21} = +18.9$ °C (c = 1, in Dimethylformamid).

## Beispiel 2

Herstellung von <Glu-His(Tos)-OH als Vergleichssubstanz für Tabelle 1

3 g (10 mmol) <Glu-His-OH werden mit 2.3 g $NaHCO_3$ in 20 ml Wasser gelöst. Dazu läßt man bei Raumtemperatur unter Rühren eine Lösung von 2.1 g (10 " Überschuß) p-Toluol-sulfochlorid in ca. 10 ml Dioxan langsam zutropfen. Nach beendigter Zugabe läßt man noch 1 Stunde nachrühren. Die Lösung wird zwei mal mit Äther ausgeschüttelt. Die wäßrige Phase wird mit 2 N HCl auf pH 2 angesäuert und der Niederschlag abgesaugt. Mit Wasser wird nachgewaschen und der Filterrückstand über $P_2O_5$ i. Vak. getrocknet. Ausbeute : 2.2 g.

## Beispiel 3

Synthese von LH-RH mit <Glu-His-OH nach verschiedenen Kupplungsmethoden gemäß Tabelle 1

640 mg H-Trp-Ser-Tyr-Gly-Leu-Arg-Pro-Gly-$NH_2$-ditosylat und 150 mg <Glu-His-OH · $H_2O$ werden in 3 ml Dimethylacetamid gelöst. Dazu gibt man 0.065 ml N-Äthylmorpholin und 68 mg HOBt (bzw. 81 mg HOObt, 90 mg HONB, 57 mg HONSu). Man kühlt auf 0 °C ab, gibt 110 mg DCC zu, läßt 1 Stunde bei 0 °C und über Nacht bei Raumtemperatur rühren. Anderntags gibt man 0.1 ml Hydrazinhydrat zu, läßt 2 Stunden bei Raumtemperatur rühren und saugt ab. Das Filtrat wird zwischen 30 ml n-Butanol und 30 ml $NaHCO_3$-Lösung verteilt. Die n-Butanolphase wird im Hochvakuum eingeengt und der Rückstand mit Äther verrieben. Der Niederschlag wird abgesaugt und getrocknet. Danach wird die Substanz in verdünnter Essigsäure gelöst und über ca. 10 ml Dowex 1 × 2 (Acetat-Form) chromatographiert. Mit Wasser wird eluiert. Das Eluat wird gefriergetrocknet. Ausbeute : 500-600 mg.

Zur weiteren Reinigung wird das Roh-LH-RH einer Verteilungschromatographie an einem hydroxypropylierten vernetzten Dextrangels analog Auslegeschrift 24 38 350, Beispiel 1 e unterworfen. Ausbeute an reinem LH-RH : ca. 250-300 mg.

## Beispiel 4

Synthese von LH-RH mit <Glu-His(Tos)-OH nach verschiedenen Kupplungsmethoden gemäß Tabelle 1

640 mg H-Trp-Ser-Tyr-Gly-Leu-Arg-Pro-Gly-$NH_2$-ditosylat und 210 mg <Glu-His(Tos)-OH werden in 3 ml Dimethylacetamid gelöst. Dazu gibt man 0,065 ml N-Äthylmorpholin und 81 mg HOObt (bzw. 57 mg HONSu). Man kühlt auf 0 °C ab, gibt 110 mg DCC zu, läßt 1 Stunde bei 0 °C und über Nacht bei Raumtemperatur rühren. Weitere Aufarbeitung und Reinigung wie bei Beispiel 3.

Ausbeute : 275 mg (bzw. 250 mg).

## Beispiel 5

Synthese von LH-RH mit <Glu-His(Dnp)-OH nach verschiedenen Kupplungsmethoden gemäß Tabelle 1

11 g (25 mmol) <Glu-His(Dnp)-OH · $0.5H_2O$ und 32 g (25 mmol H-Trp-Ser-Tyr-Gly-Leu-Arg-Pro-Gly-$NH_2$ · 2 TosOH werden in 150 ml Dimethylacetamid gelöst. Dazu gibt man 4.07 g HOObt (= 3-Hydroxy-4-oxo-3,4-dihydro-1.2.3-benzotriazin) (bzw. 3.4 g HOBt, 4.5 g HONB, 2.9 g HONSu), kühlt auf 0 °C ab und gibt dann 3.25 ml N-Äthylmorpholin und 5.5 g DCC zu. Man läßt eine Stunde bei 0 °C und über Nacht bei Raumtemperatur rühren. Der ausgefallene Niederschlag wird abgesaugt. Mit wenig Dimethylacetamid wird nachgewaschen. Das Filtrat wird mit 2.5 ml 100-proz. Hydrazinhydrat versetzt und läßt 4 Stunden bei Raumtemperatur rühren. Es entsteht eine schwarze Lösung. Mit 1 250 ml Essigester wird das Peptid gefällt. Der Niederschlag wird abgesaugt und mit Essigester gewaschen. Der Niederschlag wird in 430 ml Methanol gelöst und mit 1 400 ml Essigester das Peptid erneut gefällt. Der Niederschlag wird abgesaugt, in 1 000 ml p-Butanol gelöst und ein mal mit 1 000 ml ges. $NaHCO_3$-Lösung und dann nocheinmal mit

750 ml ges. $NaHCO_3$-Lösung ausgeschüttelt. Die n-Butanol-phase wird im Hochvakuum eingeengt. Der Rückstand wird mit Essigester verrieben und getrocknet. Rohausbeuten : ca. 25 g Weitere Reinigung analog Beispiel 3.

Ausbeute : 12-14 g.

Beispiel 6

Synthese von [D-Ser(Bu$^t$)$^6$]LH-RH-(1-9)-nonapeptid-äthylamid mit ⌐Glu-His(Dnp)-OH nach verschiedenen Kupplungsmethoden gemäß Tabelle 2

11 g (25 mmol) ⌐Glu-His(Dnp)-OH · O · $5H_2O$ und 26.6 g (25 mmol) H-Trp-Ser-Tyr-D-Ser(Bu$^t$)-Leu-Arg-Pro-NH-$C_2H_5$ · 2HCl werden in 150 ml Dimethylacetamid gelöst. Dazu gibt man 4.07 g 3-Hydroxy-4-oxo-3.4-dihydro-1.2.3-benzotriazin (= HOOBt) (bzw. 3.4 g HOBt, 4.5 g HONB oder 2.9 g HONSu) und kühlt auf 0 °C ab. Dazu gibt man 3.25 ml N-Äthylmorpholin und 5.5 g DCC zu. Man läßt eine Stunde bei 0 °C und über Nacht bei Raumtemperatur rühren. Der ausgefallene Niederschlag wird abgesaugt und mit 50 ml Dimethylacetamid gewaschen. Das Filtrat wird mit 2.5 ml 100-proz. Hydrazinhydrat versetzt. Man läßt 4 Stunden bei Raumtemperatur rühren und fällt mit 1 250 ml Essigester das Peptid aus. Der Niederschlag wird abgesaugt und gut mit Essigester gewaschen. Danacht wird die Substanz in 430 ml Methanol gelöst. Mit 1 400 ml Essigester wird das Peptid erneut gefällt. Der Niederschlag wird in 1 000 ml n-Butanol gelöst und ein mal mit 1 000 ges. $NaHCO_5$-Lösung und ein zweites mal mit 750 ml ges. $NaHCO_3$-Lösung ausgeschüttelt. Die n-Butanol-phase wird eingeengt und der Rückstand mit Essigester verrieben. Der Niederschlag wird abgesaugt, mit Essigester gewaschen und getrocknet. Ausb. 18.7 g.

Zur Überführung in das Acetat wird die oben gewonnene Substanz in 50 ml Wasser und 5 ml Essigsäure gelöst und über 230 ml Dowex 1 × 2 (Acetat-Form) chromatographiert. Mit Wasser wird eluiert. Die Fraktionen, die Substanz enthalten, werden vereinigt und gefriergetrocknet. Ausbeute : ca. 15.0 g

$[\alpha]_D^{23} = -40$ °C (c = 1, in Methanol)

Weitere Reinigung analog Beispiel 3.

Ausbeute : ca. 9.5-11 g.

Beispiel 7

Synthese von [D-Ser(Bu$^t$)$^6$]LH-RH-(1-9)-nonapeptid-äthylamid mit ⌐Glu-His-OH nach verschiedenen Kupplungsmethoden gemäß Tabelle 2

532 g H-Trp-Ser-Tyr-D-Ser(Bu$^t$)-Leu-Arg-Pro-$NHC_2H_5$ · 2HCl und 150 mg ⌐Glu-His-OH · $H_2O$ werden in 3 ml Dimethylacetamid gelöst. Dazu gibt man 0.065 ml n-Äthylmorpholin und 68 mg HOBt (bzw. 81 mg HOOBt, 90 mg HONB oder 57 mg HONSu). Man kühlt auf 0 °C ab, gibt 110 mg DCC zu, läßt 1 Stunde bei 0 °C und über Nacht bei Raumtemperatur rühren.

Aufarbeitung und Reinigung analog Beispiel 3.

Ausbeute : 104-207 mg.

**Ansprüche** (für die Vestragsstaaten : BE, CH, DE, FR, GB, IT, LU, NL, SE)

1. Verfahren zur Herstellung von LH-RH bzw. LH-RH-Analoga, dadurch gekennzeichnet, daß man ⌐Glu-His(Dnp)-OH mit dem entsprechenden Peptid mit freier Amino- und geschützter Carboxylgruppe in einem in der Peptidchemie üblichen Lösungsmittel unter Zusatz von 3-Hydroxy-4-oxo-3.4-dihydro-1.2.3-benzotriazin (HOObt) und einem Carbodiimid umsetzt und anschließend die Dinitrophenylgruppe abspaltet.

2. Verfahren nach Anspruch 1, dadurch gekennzeichnet, daß die Abspaltung der Dnp-Schutzgruppe mit Hydrazin oder Thiolen erfolgt.

3. Dipeptid ⌐Glu-His(Dnp)-OH.

**Ansprüche** (für den Vertragsstaat AT)

1. Verfahren zur Herstellung von LH-RH bzw. LH-RH Analoga, dadurch gekennzeichnet, daß man ⌐Glu-His(Dnp)-OH mit dem entsprechenden Peptid mit freier Amino- und geschützter Carboxylgruppe in einem in der Peptidchemie üblichen Lösungsmittel unter Zusatz von 3-Hydroxy-4-oxo-3.4-dihydro-1.2.3-benzotriazin (HOObt) und einem Carbodiimid umsetzt und anschließend die Dinitrophenylgruppe abspaltet.

2. Verfahren nach Anspruch 1, dadurch gekennzeichnet, daß die Abspaltung der Dnp-Schutzgruppe mit Hydrazin oder Thiolen erfolgt.

**Claims** (for the Contracting States : BE, CH, DE, FR, GB, IT, LU, NL, SE)

1. Process for the manufacture of LH-RH and LH-RH analogs, which comprises reacting ⸢Glu-His(Dnp)-OH with the respective peptide having a free amino group and protected carboxy group in a solvent as used in peptide chemistry with the addition of 3-hydroxy-4-oxo-3,4-dihydro-1,2,3-benzotriazine (HOObt) and a carbodiimide and subsequently splitting off the dinitrophenyl group.

2. The process of claim 1, wherein the Dnp-protective group is split off by means of hydrazine or a thiol.

3. The dipeptide ⸢Glu-His(Dnp)-OH.

**Claims** (for the Contracting State AT)

1. Process for the manufacture of LH-RH and LH-RH analogs, with comprises reacting ⸢Glu-His(Dnp)-OH with the respective peptide having a free amino group and protected carboxy group in a solvent as used in peptide chemistry with the addition of 3-hydroxy-4-oxo-3,4-dihydro-1,2,3-benzotriazine (HOObt) and a carbodiimide and subsequently splitting off the dinitrophenyl group.

2. The process of claim 1, wherein the Dnp-protective group is split off by means of hydrazine or a thiol.

**Revendications** (pour les Etats contractants : BE, CH, DE, FR, GB, IT, LU, NL, SE)

1. Procédé de préparation de LH-RH ou d'analogues du LH-RH, caractérisé en ce qu'on fait réagir ⸢Glu-His(Dnp)-OH avec le peptide correspondant ayant son groupe amino libre et son groupe carboxyle protégé dans un solvant habituellement utilisé dans la chimie des peptides avec addition de 3-hydroxy-4-oxo-3,4-dihydro-1,2,3-benzotriazine (HOObt) et un carbodiimide, puis on élimine le groupe dinitrophényle.

2. Procédé suivant la revendication 1, caractérisé en ce que l'élimination du groupe protecteur Dnp est effectuée avec de l'hydrazine ou des thiols.

3. Dipeptide ⸢Glu-His(Dnp)-OH.

**Revendications** (pour l'Etat contractant AT)

1. Procédé de préparation de LH-RH ou d'analogues du LH-RH, caractérisé en ce qu'on fait réagir ⸢Glu-His(Dnp)-OH avec le peptide correspondant ayant son groupe amino libre et son groupe carboxyle protégé dans un solvant habituellement utilisé dans la chimie des peptides avec addition de 3-hydroxy-4-oxo-3,4-dihydro-1,2,3-benzotriazine (HOObt) et un carbodiimide, puis on élimine le groupe dinitrophényle.

2. Procédé suivant la revendication 1, caractérisé en ce que l'élimination du groupe protecteur Dnp est effectuée avec de l'hydrazine ou des thiols.